Europäisches Patentamt

(19) European Patent Office

Office européen des brevets

(11) Publication number : **0 594 176 A1**

(12) **EUROPEAN PATENT APPLICATION**

(21) Application number : 93117093.0

(22) Date of filing : 21.10.93

(51) Int. Cl.⁵ : **A61B 17/04, A61B 17/58, A61F 2/02**

(30) Priority : 23.10.92 US 966130

(43) Date of publication of application :
27.04.94 Bulletin 94/17

(84) Designated Contracting States :
DE FR GB IT

(71) Applicant : **United States Surgical Corporation**
**150 Glover Avenue**
**Norwalk, Connecticut 06856 (US)**

(72) Inventor : **Chesterfield, Michael P.**
**32 Bayne Street**
**Norwalk, CT 06851 (US)**
Inventor : **Liu, Cheng-Kung**
**2 Minerva Street**
**Norwalk, CT 06851 (US)**
Inventor : **Koyfman, Ilya**
**420 Miles Road**
**Orange, CT 06477 (US)**

(74) Representative : **Marsh, Roy David et al**
**Hoffmann Eitle & Partner, Patent- und**
**Rechtsanwälte, Arabellastrasse 4**
**D-81925 München (DE)**

(54) **Surgical repair product.**

(57) Textile surgical articles (10) are disclosed which are constructed in whole or in part from high tenacity low elongation absorbable fibers. The products may be braided, woven or knitted, such as braided tapes. The high tenacity low elongation absorbable fibers provide structures having greatly increased strength and decreased elongation, a combination of properties which is uniquely applicable and superior for repairing body tissue (12, 14).

FIG. 1

EP 0 594 176 A1

## FIELD OF THE INVENTION

The present invention relates to products for surgical repair of body tissue. In particular, the invention is directed to reinforced surgical repair products for repairing the human sternum after surgery.

## BACKGROUND OF THE INVENTION

Presently there are many known products for repairing human body tissue in areas where a repair may be required either as a result of an injury or during or after surgery. In particular, it is well known to utilize suture products in the form of elongated strands to repair human body tissue as well as utilizing two-part fasteners or metal staples for attaching body tissue after portions have been removed during surgery.

For example, sutures intended for repairing soft body tissue are usually constructed of a plurality of filaments and applied to the tissue with any number of surgical needles. More recently, a certain amount of emphasis has been placed upon repairing bone utilizing an elongated surgical product in the form of a flat band. A needle is used to penetrate and/or go around the bone and to apply the repair product to the bone in a manner which physically retains the separated bone portions together to promote permanent healing. One such example is disclosed in U.S. Patent No. 4,535,764 to Ebert which relates to a surgical bone tie having a needle connected to one end of a band such that the band may be looped and arranged to be appropriately looped around the bone portions requiring repair.

U.S. Patent No. 4,813,416 relates to a band assembly and method for sternum closing with which the sternum halves are brought to abutting closure utilizing a band having a needle at one end to facilitate looping the band in position to retain the sternum portions in adjacent butting contacting relation.

Numerous other products have been used to retain bone portions together to promote healing while numerous suture products have been used to retain soft tissue to retain healing.

The physical strength characteristics of the components which form the band product must provide the surgeon with precision control of the product. Moreover, the product should not cause unnecessary cutting of the tissue when force is applied to the product and that force is in turn applied to the tissue.

A particularly desirable product for accomplishing these goals would preferably display substantial strength without significant elongation to facilitate retaining the tissue portions together. In the case of attaching separate bone portions of the sternum together after open heart surgery for example, it has been necessary to utilize stainless steel wire by looping the wire around the sternum portions and actually twisting the ends together to form an attachment. The metal wire displayed sufficient strength to retain the bone portions together without elongation. However, the wire represented a relatively sharp non-absorbable foreign body which remains embedded within the body tissue and thus presents a potential source of infection or other complications as a result of its presence within the body. Moreover, the relatively small wire diameter generates stress over a small area presenting a danger of cutting into the bone during the application to the sternum. The sharp wire also presents a hazard to the surgeon and operating room personnel in that the wire may penetrate surgical gloves and cut the surgeon or attendant personnel, especially during CPR thereby creating a potential site for transmission of disease.

While utilization of wire sutures has been used and accepted during open heart surgery there remains room for improvement in the products used for strapping the split sternum portions together. Desirably, it would be best to provide a product which not only provides adequate strength and elongation characteristics but which may be utilized to form a tying product for soft as well as hard tissue, in a manner which will minimize the dangers of cutting of the tissue in the surrounding areas. It would also be desirable to provide a product which could be broken down by the body into metabolizable, non-toxic components; i.e., a bioabsorbable product. The present invention is directed to such a product.

## SUMMARY OF THE INVENTION

In accordance with the present invention, textile surgical articles are disclosed which are made in whole or in part from high tenacity, low elongation absorbable fibers such as fibers of glycolide-lactide copolymers having a high lactide content. Particularly useful copolymers contain between about 70 and 95 mole percent lactide. The products may be braided, woven or knitted. The high tenacity low elongation fibers provide structures having greatly increased strength and decreased elongation.

In a preferred method of the invention, a braided tape made of high tenacity absorbable fibers is used to join a divided sternum by tying, or other appropriate means. The tape has a very high strength, preferably equal to or greater than 35 kg. straight-pull strength, and elongation at break below about 25%, preferably below about 20%.

## BRIEF DESCRIPTION OF THE DRAWINGS

Preferred embodiments of the invention are described hereinbelow wherein:

Fig. 1 is a perspective view of a portion of a split human sternum illustrating one application of the present invention for retaining the split portions together to promote healing;

Fig. 2 is an enlarged view of the product shown in Fig. 1 illustrating one embodiment wherein the elongated product is a flat braided member and contains eight reinforcing filaments extending along the length;

Fig. 3 is a cross-sectional view taken along lines 3-3 of Fig. 2.;

Fig. 4 is an enlarged view of an alternative embodiment of the suture repair product of Fig. 2 wherein the elongated braided product contains at least seven reinforcing filaments extending along the length; and

Fig. 5 is a cross-sectional view taken along lines 5-5 of Fig. 4.

## DETAILED DESCRIPTION OF THE PREFERRED EMBODIMENTS

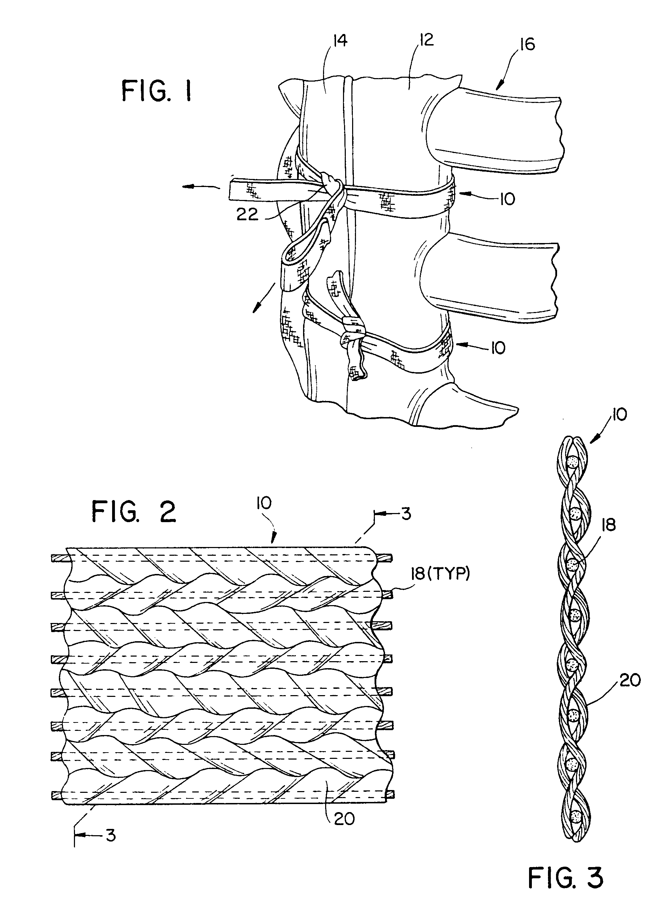

Referring initially to Fig. 1 there is illustrated a sternum closure ribbon 10 constructed according to the present invention and positioned to retain portions 12,14 of a human sternum 16 together. The band 10 is preferably a braided product as shown in Fig. 2 having a plurality of elongated filamentary reinforcing members 18. The reinforcing fibers are made of an absorbable copolymer which contains greater than 50 mole percent lactide. Preferably, the fibers 18 are made from a copolymer containing at least 70% lactide. Most preferably, the copolymer contains about 80 mole percent lactide. The fibers may be coated or otherwise treated to improve slip characteristics of the yarn, if desired. Suitable coatings include polyethylene oxide-polypropylene oxide block copolymer or polyalkylene glycol, either of which has been further polymerized with lactide monomer or glycolide monomer.

The high tenacity low elongation absorbable fibers employed in the present invention are formed from polymers preferably having a molecular weight such that the inherent viscosity (when measured in chloroform at 30° C in a concentration of about 2.5 g/dl) of the polymer is between about 1.0 and 1.7 dl/g and preferable between 1.3 and 1.5 dl/g. The polymers exhibit a very substantial degree of crystalline orientation and crystalline content. As a result the fibers exhibit tensile strengths from about 70 kpsi (thousands of pounds per square inch) to about 115 kpsi and tensile moduli of from about 0.85 msi (millions of pounds per square inch) to about 1.28 msi. The significant strength and stability of these fibers are caused by the high degree of molecular orientation. The copolymers used in accordance with this invention will therefore have sufficiently long absorption times and strength to be useful in the sternum closure application. Moreover, since the fibers can be provided as multifilament or monofilament fibers which can be braided, woven, knitted or otherwise processed to form a textile product it will be readily appreciated that any number of reinforced textile products may be provided similar to the band 10 shown in the drawings, but with numerous alternative structures including those described hereinbelow.

Referring now to Fig. 2, the band 10 shown in Fig. 1 is shown in greater detail as an elongated flat braided textile product having a plurality of fibers 18 extending along the length of the band.

Fibers 20 which make up the fill yarns may be made of any suitable bioabsorbable polymeric material such as polymers or copolymers of glycolide, lactide, p-dioxanone, polyester, polyamino acids and the like as disclosed in U.S. Patent Nos. 2,668,162; 3,297,033; 3,636,956; 3,736,646; and 3,839,297. Preferably, the fill yarns are fibers made of the same copolymer as the reinforcement fibers 18.

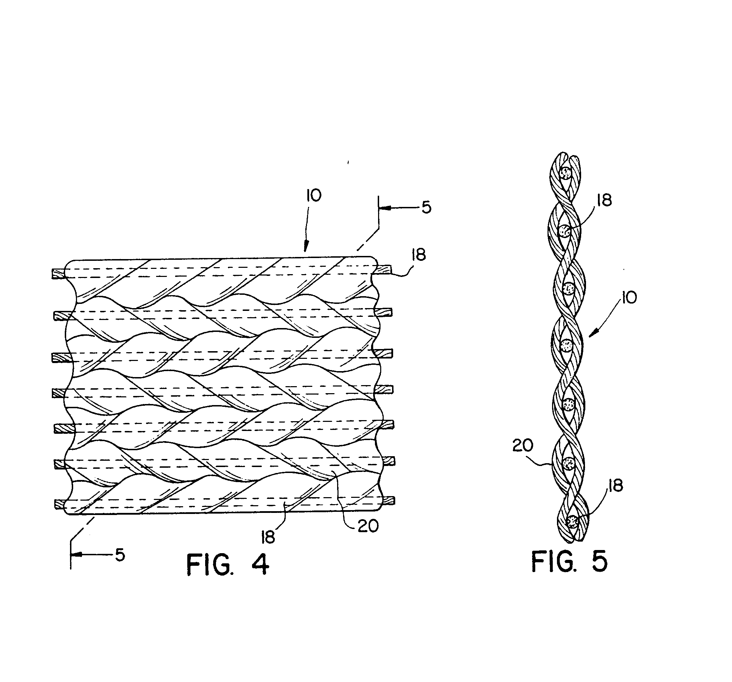

The number of reinforcing filaments 18 included in the braided band 10 shown in Fig. 2 is a matter of choice as is the specific construction of the band. For example, as seen in Fig. 4, there is an example of an alternative braided band construction having seven reinforcing filaments 18 of high strength fibers of the type shown in Fig. 2. Any number of combinations of bio-absorbable yarns, filamentary or otherwise, are contemplated, depending upon the intended application.

It will be appreciated that in addition to the examples which follow hereinbelow, numerous alternative textile constructions may be incorporated into the present invention to form a reinforced band for attaching body tissue such as a soft tissue or bone tissue without suffering from the disadvantages of presently known materials. For example, it is conceivable within the scope of the present invention to provide a woven structure containing a plurality of elongated high strength absorbable filaments 18 in the warp direction wherein the filler yarns are of another suitable bioabsorbable material such as polymers or copolymers of glycolide, lactide, p-dioxanone, polyester, polyamino acids and the like. Likewise, knitted structures may be strengthened by reinforcement with high tenacity absorbable fibers. It will be appreciated that in each of the embodiments discussed herein the strength characteristics of the high tenacity, low elongation absorbable fibers 18 will provide the substantial force carrying capability to the elongate product while the fibers 20 surrounding the high strength filaments will provide the necessary structural support to the main fibers for forming the product. The surrounding fibers will also define the "hand" or "feel" of the band.

Accordingly, it is possible in one application to position the reinforced structure 10 about the split portions 12, 14 of the human sternum 16 as shown in Fig. 1 whereby substantial force may be applied to the band by tying the band either by a knot 22 shown in Fig. 1, or by other techniques whereby significant force may be applied and retained to promote natural healing of the sternum portions 12,14, e.g. mechanical connecting devices such as buckles, etc. See, for example, U.S. Patent No. 4,813,416. The strength and load carrying capability of the elongated absorbable filaments 18 is sufficient to transmit substantial force to the sternum with minimum elongation occurring to the fibers thereby permitting the sternum portions to undergo a natural healing process. Filaments 18 retain their strength in vivo for a period of time sufficient to allow healing of the bone or tissue. Preferably, the fibers retain at least about 15% of their strength after implantation in the body for twelve weeks. Most preferably, the fibers retain from about 30 to about 45% of their strength after implantation in the body for twelve weeks. Furthermore, in addition to the textile processes of braiding and weaving it should be noted that alternative textile processes may be utilized including knitting techniques, provided that the final product contains a plurality of elongated high strength filaments 18 extending along at least the length of the product in the force-carrying direction to maintain the tissue portions together.

The product will be manufactured to include a plurality of high strength, high tenacity absorbable filaments as disclosed hereinabove, either as a component of the product, e.g. a core or reinforcing fiber, or as the sole material used to construct the product. The fibers may be air entangled periodically to create a false twist before incorporation into a braided, woven or knitted structure. It is contemplated that the yarn could instead be twisted prior to braiding or other operation, with all or some of the yarn twisted in each of the "s" or "z" directions. In addition, the yarn and/or product may be coated or treated depending upon the particular needs or intended application so as to reduce the perceived "slipperiness" of the product as desired.

The final product could be provided with a surgical needle at one end to facilitate insertion of the product into the body tissue whether the body tissue be soft skin tissue or hard bone tissue, or the needle may be utilized to facilitate looping the product into and out of spaces formed between the component members of the body such as the components forming the human sternum. Alternatively, the product could be provided with a needle at each end to facilitate ease of application to the body portions. In either event, the strength and the load carrying filaments 18 and the minimal elongation to strength percentage renders such filaments ideal for incorporation into a final product wherein body portions can be retained together to promote healing. In particular, the formation of a surgical repair product utilizing textile processes in combination with other bioabsorbable filaments renders the incorporation of high tenacity, low elongation, absorbable filaments 18 as an ideal combination to form a surgical suture repair product.

The following examples show the preparation of a copolymer suitable for use in the present invention and a flat tape which can be utilized to tie two half portions of a human sternum to promote healing. Braiding of the tape with yarns of the particular copolymer of Example 1 is noted for exemplary purposes only and other suitable bioabsorbable or nonabsorbable yarns may be appropriately substituted, as desired or appropriate for a particular construction. Of course, substitution of different yarns may require variations to the structure as required to accommodate changes in density and/or fiber denier.

## EXAMPLE 1 - PREPARATION OF COPOLYMER

A copolymer of glycolide and lactide is prepared as follows:

Hydroxyacetic acid (glycolic acid) is heated under nitrogen to 180° C to remove impurities such as water. Pressure is then reduced and heating is continued for two hours to yield a prepolymer of polyglycolic acid, which is recovered and powdered.

The prepolymer is heated in the presence of $Sb_2O_3$ at 275° C under low pressure with an argon purge and stirring. The prepolymer cracks and glycolide is distilled over and recovered in a cold vacuum receiver. Preferably, the glycolide is purified by conventional techniques, such as distillation, crystallization, and sublimation.

L-lactide is used alone or in combination with a small amount of the DL racemer. L-lactide is purified by crystallization from toluene solution. The DL racemer, if used, is purified by crystallization from ethyl acetate.

A mixture of the purified glycolide (18 mole percent) and lactide (82 mole percent) is charged to a reactor under an argon blanket. A solution of stannous octoate catalyst in diethyl ether is added to give 0.92% w. of catalyst, based on the total weight of glycolide and lactide. The reactor is further purged with argon and held at 5 psi while heating to 170° - 175° C. Pressure and temperature are maintained for six hours.

The reaction product is isolated, comminuted, and treated to remove residual reactants. Any method capable of removing the unreacted monomers from the crude reaction product may be used. A preferred purification procedure is as follows.

After comminution, the crude reaction product is contacted with ethyl ether for about 72 hours in a Soxhlet-type extractor to remove unreacted monomer. Typically, 4-10% of the starting monomers remain unreacted, and the glass transition temperature of the crude copolymer is approximately 50° C. Removal of unreacted monomers raises the glass transition temperature. As will be understood by one skilled in the art, the composition of the copolymer may differ slightly from the composition of the starting monomeric mixture because the lactide and glycolide are not of equal reactivity.

After the extraction period, the partially purified copolymer is slowly heated under vacuum from ambient temperature to 140° C over a period of about 48 hours. The slow rate of heating is desirable to prevent melting (strictly speaking, flowing together) of the copolymer particles and to remove any water present. Desirably, dry inert gas is used to purge the system, and occasionally the heating step may require more than 48 hours to reach the desired glass transition temperature. The combination of slow heating and purging with dry gas removes any residual solvent (ethyl ether) present, thereby raising the glass transition temperature.

After removal of unreacted monomers (and of solvent, if solvent extraction is used), the purified copolymer is further dried if it was not dried enough in the monomer removal step and, in any event, stored to keep it dry.

## EXAMPLE 2

A braided tape having multifilament runners (45 filaments, 90 denier) and fill yarns was made on a 17 carrier braider with 8 parallel runners with all yarns being made from the copolymer of Example 1. This structure is shown in Figs. 2 and 3. The fill yarns were made with five plies of air entangled 90 denier, 45 filament yarn. The properties of the tape were measured as follows:

| | |
|---|---|
| Denier | 12,620 |
| Tape Thickness | .485 mm |
| Tape Width | 4mm |
| Knot-pull strength | 24.5 kg |
| Straight-pull strength | 37.3 kg |
| Elongation at break | 18.5% |
| Pick count | 3 crossovers per inch |

The claims which follow identify embodiments of the invention additional to those described in detail above.

## Claims

1. A braided surgical product for surgical repair which comprises a flexible elongate member formed at least in part of high tenacity absorbable fibers.

2. A braided surgical product adapted to be looped about split portions of human tissue to retain the tissue portions in juxtaposed relation, which comprises a flexible elongate member reinforced with high tenacity absorbable fibers.

3. The product according to claim 1 or 2 wherein said elongate member has an elongation to break below about 25 percent.

4. A surgical repair product adapted to be looped about split portions of the human sternum to retain the split portions in adjacent contacting relation to promote healing, which comprises an elongate member formed at least in part of a plurality of elongated fibers, said fibers being of high tenacity absorbable copolymer, said fibers having an elongation to break of approximately twenty percent or less.

5. The product according to any one of the preceding claims wherein said elongate member is of flat braided construction.

6. The product according to any one of the preceding claims wherein said elongate member contains a plurality of said high tenacity absorbable fibers extending along the length thereof.

7. The product according to claim 6 as dependent on claim 5 wherein said flat braided member contains at least about 7 of said high tenacity absorbable fibers extending along the length thereof.

8. The product according to any one of the preceding claims wherein said high tenacity absorbable fibers comprise a copolymer containing at least 50 mole percent lactide.

9. The product according to any one of the preceding claims wherein said high tenacity absorbable fibers comprise a random copolymer containing between about 70 and about 95 mole percent lactide.

10. The surgical product according to any one of the preceding claims wherein said elongate member comprises a band of woven construction formed at least in part from said absorbable fibers extending in the warp direction.

11. The surgical product according to any one of the preceding claims having absorbable copolymer fibers extending generally in the weft direction.

12. The surgical product according to claim 11 having fibers of a copolymer comprising between about 70 and about 95 mole percent lactide extending generally in the weft direction.

13. The surgical product according to claim 9 wherein said elongate member includes bioabsorbable polymeric filler yarns selected from polymers or copolymers of glycolide, lactide, p-dioxanone, polyester, polyamino acids, trimethylene carbonate and epsilon-caprolactone.

14. The surgical product according to any one of the preceding claims wherein said elongate member has a straight-pull strength greater than about 35 kg.

FIG. 1

14
12
16

22

10

10

FIG. 2

10

3

18(TYP)

20

3

FIG. 3

10

18

20

FIG. 4

FIG. 5

**European Patent Office**

# EUROPEAN SEARCH REPORT

Application Number

EP 93 11 7093

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 239 775 (AMERICAN CYANAMID COMPANY) | 1-7,10, 11,14 | A61B17/04 A61B17/58 |
| Y | * page 5, line 14-21; figures * | 8,9,12, 13 | A61F2/02 |
| | * page 7, line 13-25 * --- | | |
| D,Y | US-A-3 736 646 (AMERICAN CYANAMID COMPANY) | 8,9,12, 13 | |
| | * the whole document * --- | | |
| A | US-A-4 959 069 (BRENNAN) --- | | |
| A | US-A-4 141 087 (SHALABY) ----- | | |

TECHNICAL FIELDS
SEARCHED      (Int.Cl.5)

A61B
A61F

The present search report has been drawn up for all claims

| Place of search | Date of completion of the search | Examiner |
|---|---|---|
| THE HAGUE | 1 February 1994 | Steenbakker, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)